# EUROPEAN PATENT SPECIFICATION

(11) **EP 1 499 890 B1**
(45) Date of publication and mention of the grant of the patent: **28.04.2010**
(21) Application number: 03736555.8
(22) Date of filing: 07.05.2003
(51) Int. Cl.: G01N 33/53

(54) **High throughput method to identify biomolecule delivery formulations**
"High througput" Methode, um Biomolekülanlieferung Formulierungen zu kennzeichnen
Méthode à haut débit pour l'identification de formulations permettant la délivrance de biomolécules

(30) Priority: 07.05.2002 US 378709 P
(43) Date of publication of application: 26.01.2005
(73) Proprietor: THE RESEARCH FOUNDATION OF STATE UNIVERSITY OF NEW YORK, Amherst, NY 14228-2567 (US)
(72) Inventor: BRIGHT, Frank, V., Williamsville, NY 14221 (US); GARDELLA, Joseph, A., Buffalo, NY 14216-2037 (US); HARD, Robert, Williamsville, NY 14221 (US); HICKS, Wesley, L., Jr., Angola, NY 14006 (US)
(74) Representative: MacLean, Martin Robert
(86) International application number: PCT/US2003/014189
(87) International publication number: WO 2003/095515

(56) References cited:
- WO-A-02/35241
- GERRITSEN M ET AL: "Biocompatibility evaluation of sol-gel coatings for subcutaneously implantable glucose sensors" BIOMATERIALS, ELSEVIER SCIENCE PUBLISHERS BV., BARKING, GB, vol. 21, no. 1, 2000, pages 71-78, XP002978566 ISSN: 0142-9612
- BENOIT M-A ET AL: "Antibiotic-loaded plaster of Paris implants coated with poly lactide-co-glycolide as a controlled release delivery system for the treatment of bone infections" INTERNATIONAL ORTHOPEADICS, SPRINGER, BERLIN, DE, vol. 21, no. 6, 1997, pages 403-408, XP002978565 ISSN: 0341-2695
- LUNDGREN J.S. ET AL: 'Dynamics of Acrylodan-Labeled Bovine and Human Serum Albumin Sequestered within Aerosol-OT Reverse Micelles' ANAL. CHEM. vol. 67, 1995, pages 3775 - 3781, XP002978563
- JORDAN J.D. ET AL: 'Dynamics of Acrylodan-Labeled Bovine and Human Serum Albumin Entrapped in a Sol-Gel-Derived Biogel' ANAL. CHEM. vol. 67, 1995, pages 2436 - 2443, XP002978564
- ARENKOV P. ET AL: 'Protein Microchips: Use for Immunoassay and Enzymatic Reactions' ANAL. BIOCHEM. vol. 278, no. 2, February 2000, pages 123 - 131, XP002271989
- BENOIT MA ET AL: 'Antibiotic-loaded plaster of Paris implants coated with poly lactide-co-glycolide as a controlled release delivery system for the treatment of bone infections' INT. ORTHOP. vol. 21, no. 6, 1997, pages 403 - 408, XP002978565
- GERRITSEN M. ET AL: 'Biocompatibility evaluation of sol-gel coatings for subcutaneously implantable glucose sensors' BIOMATERIALS vol. 21, no. 1, 2000, pages 71 - 78, XP002978566
- ZENTNER G M; RATHI R; SHIH C; MCREA J C; SEO M H; OH H; RHEE B G; MESTECKY J; MOLDOVEANU Z; MORGAN M; WEITMAN S: "Biodegradable block copolymers for delivery of proteins and water-insoluble drugs" JOURNAL OF CONTROLLED RELEASE : OFFICIAL JOURNAL OF THE CONTROLLED RELEASE SOCIETY, vol. 72, no. 1-3, 14 May 2001 (2001-05-14), pages 203-215, XP004246450 Netherlands

## Description

Biodegradable polymers are the center piece of numerous biomedical devices (e.g., sutures and temporary implants). Synthetic biodegradable polymers have also been used as a matrix in clinical applications for decades. Some relevant applications include surgical implants, wound healing materials, and drug delivery devices. Other polymeric materials as well as sol-gel-derived materials such as bioceramics and bioglasses, have been identified as useful implantable matrices for delivery of biomolecules.

Sol-gel-derived materials are also promising platforms for the development of chemical sensors and biosensors as well as providing materials which can be implanted in a body. Xerogels are substances made up of microscopic glass bubbles filled with air and are formed when the liquid part of an alcogel (wet gel) is removed by evaporation or similar methods. The microscopic bubbles provide sites for the entrapment of biomolecules when the xerogels are doped with biomolecule. However, despite significant progress in these materials, there has not previously been a method of rapidly preparing and screening formulations wherein a significant proportion of the biomolecule(s) contained within the formulation is present in its functional chemical structure and/or delivered from said structure in its functional chemical structure.

There are numerous reviews by leading bioengineering groups on the formulation of protein growth factors and other proteinaceous species within biodegradable polymers for use as drug delivery vehicles or as a tissue scaffold to grow a biologic/biomaterial composite for later (re-)implantation. For example, it has been reported that insulin like growth factor-I (IGF-I) and transforming growth factor-β1(TGF-β1) for bone morphogenesis can be delivered from a poly(D,L-lactide) (PDLLA)-coated implant This was accomplished by the preparation of solutions of protein and PDLLA directly suspended/dissolved in chloroform and then formed as films on the implants by dip casting. An enzyme linked immunosorbent assay was used to determine the protein concentration that was liberated/released from these protein-doped films. Although this loading method appears to yield formulations that can deliver active TGF-β1 and IGF-I, the fraction of total protein that was released and that was active was much less than unity. Furthermore, there is ample literature demonstrating that organic solvents can have a negative effect on protein association and function. See, e.g., Klibanov, AM, Nature; 409:241-246 (2001); Klibanov, AM, Trends Biotechnol; 15:97-101 (1997); Constantino, HR, et al., J Pharm Sci; 83:1662-1669 (1994); and Wescott, CR, et al., Biochim Biophys Acta; 1206:1-9 (1994).

Methods used to prepare medical devices from matrix materials can involve the use of organic solvents as casting solvents. While effective in solubilizing certain matrix materials, casting solvents can have a negative, denaturing effect on biomolecule(s) that may be added to the formulations. Also, when the formulations contain biodegradable polymers, the degradation products of the polymer may interact with the biomolecule to diminish the functionality of the biomolecule. For example, lactic acid generated by the degradation of lactide containing polymers can disrupt the functionality of a biomolecule.

Additionally, the need for biocompatibility within an implant site has been recognized. However, the key issue of guided tissue response, such as an increased and sustained rate of reepithelialization to reestablish lumenal integrity while discouraging the formulation of granulation tissue, obtained through the use of a medical device comprising a formulation that is composed of a biomolecule-doped implantable matrix, wherein a significant proportion of the biomolecule material present in the formulation is present in its functional chemical structure, has not been addressed until now.

Antibodies are large multi-domain proteins that bind to antigens/haptens with good selectivity and high affinity (to 10¹⁰ M⁻¹). These features have attracted numerous researchers to develop biosensors that are based on immobilized antibodies. The behavior of biomolecule materials including antibodies sequestered within sol-gel derived xerogels to design biosensors with desirable analytical figures of merit has been investigated. However, a major impediment to these efforts has been the need to investigate a wide range of precursors, codopants, and compositions to gain insight into the factors that control the sequestered material's function within xerogels. Immobilized biomolecules, e.g., antibodies, have also been identified as useful for implantable medical devices, e.g., vertebral implants.

Thus, in both arenas (i.e., doped polymeric and sol-gel formulations) there is a need to rapidly prepare and screen formulations containing a biomolecule in its functional chemical structure and identify those formulations that exhibit or are the most likely to exhibit the best performance. (In this context, best performance involves providing a biomolecule containing formulation having a significant proportion of the biomolecule in its functional chemical structure; be that as a medical device or as a sensor platform.)

High-throughput screening (HTS) methods are common in combinatorial chemistry and drug discovery. HTS methods have been used to identify promising H₂-producing catalytic materials, ceramics, flame retardants, dielectric thin films, and other materials. However, prior to the disclosure contained herein, it was not appreciated that HTS methods could be applied to biodegradable polymer formulations and sol-gel formulations. For example, if the biodegradable polymer formulations and sol-gel formulations one wishes to test is at some point liquidous, one can use pin-printing methods to print a biomolecule-doped material into an array and screen the array to identify lead compositions that are able to maintain a significant proportion of the biomolecule material in its functional chemical structure.

WO 02/35241 describes high-throughput screening for protein formulations.

Gerritsen M. et al., Biomaterials, Vol. 21, No. 1, 2000, pages 71-78, describes biocompatibility evaluation of sol-gel coatings for subcutaneously implantable glucose sensors.

Benoit M-A. et al., International Orthopedics, Vol. 21, No. 6, 1997, pages 403-408, describes antibiotic-loaded plaster of Paris implants coated with polylactide-co-glycolide as a controlled release delivery system for the treatment of bone infections.

Zentner G M et al., Journal of Controlled Release, Vol. 72, No. 1-3, 2002, pages 203-215, describes biodegradable block copolymers for delivery of proteins and water-insoluble drugs.

To address these needs, we disclose high-speed production and screening methods to identify biomolecule-doped formulations that can contain a significant proportion of biomolecule in its functional chemical structure. The improvement in containing biomolecules wherein a significant proportion of the biomolecule is in its functional chemical structure is significantly enhanced by the presence in the formulation of a protectant material, such as a surfactant capable of forming reverse micelles or ion-containing polymers that can form protective domains. This is an important step toward finding novel materials that can be used as biosensors or medical devices.

The present invention thus provides a high throughput method of identifying one or more biomolecule delivery formulations, said method comprising the steps of:
(a) providing a plurality of different test formulations, each formulation prepared by a method comprising:
   (i) a biomolecule, wherein the biomolecule has an intrinsic luminescence emission spectrum, and wherein the biomolecule is selected from the group consisting of peptides, polypeptides and proteins,
   (ii) contacting the biomolecule with a protectant material, wherein the protectant material is a surfactant, and
   (iii) sequestering the biomolecule from (ii) in a matrix material, wherein the matrix material is a sol-gel derived glass or a biodegradable polymer;
(b) creating an array of the test formulations;
(c) recording a luminescence emission spectrum for each test formulation in the array;
(d) comparing the luminescence emission spectrum from each test formulation in the array to a luminescence emission spectrum from a control formulation, wherein the control formulation has the biomolecule present in a native conformation; and
(e) identifying one or more formulations as biomolecule delivery formulations, based upon comparison of said luminescence emission spectrum from each test formulation to said luminescence emission spectrum from the control formulation.
(f) preparing thin-film specimens of each of the one or more candidate biomolecule delivery formulations; and
(g) determining the release kinetics of biologically active labelled biomolecules from each of the one or more thin-film specimens, wherein the release of biologically active labelled biomolecules identifies a biomolecule delivery formulation.

Also disclosed is a new method to rapidly produce and screen identifying formulations useful as biosensors by practicing the steps of (a) providing a plurality of test formulation comprising a holding material, biosensor molecule, and a protectant material; (b) creating an array of the test formulation; (c) contacting the array with target molecules wherein the reaction of the target molecule with the biosensor produces a detectable fluorescence response upon excitation; (d) measuring the fluorescence response of each formulation in the array; and (e) identifying the formulation displaying a desired amount of the fluorescence response.

Also disclosed is a new method to rapidly produce and screen a wide variety of polymer-, xerogel-, bioceramic-, or bioglass-based formulations having a significant proportion of the biomolecule material delivered from within the formulation in its functional chemical structure. These formulations are useful in, for example, medical devices to promote a guided tissue response at an implantation site (e.g., rapid and sustained reepithelialization within the human large conducting airway, LCA) by the choreographed release of active proteins, nutrients, and essential amino acids that promote respiratory epithelial cell migration and proliferation.

Further disclosed herein is a biosensor composition including a matrix material, a biosensor molecule; and a protectant material.

The screening method of this disclosure may be based on a stand alone high-speed pin printer and an epi-fluorescence microscope with a charge coupled device (CCD) detector. The utility of such apparatuses for screening formulations is demonstrated herein by, for example, an investigation of a series of biodegradable polymer formulations that contain keratinocyte growth factor (KGF) as well as an investigation of sol-gel derived xerogels that are doped with monoclonal anti-fluorescein antibodies. Although the utility of the screening method disclosed herein is demonstrated with certain exemplary formulations of materials, it should be understood the screening method disclosed herein may be applied to any formulation capable of being printed and having a biomolecule with a detectable functional chemical structure as well as a detectable deformation or diminishment of the detectable functional chemical structure. Therefore, the examples disclosed herein as well as the materials used in preparing the formulations for screening should be viewed as non-limiting examples and are not intended to limit the scope of the claims.

Also disclosed herein are biodegradable polymeric formulations useful in providing a medical device to, for example, aid lumenal restitution. The medical device provides an engineered approach to guided tissue response. Such a guided response may include, for example, providing a temporary barrier function against the unregulated growth of tissue (e.g., granulation tissue) or unregulated secretion of proteins that are inhibitory to reepithelialization; optionally supporting cell adhesion, migration and proliferation; and/or delivering active cytokines/growth factors and other species that promote desirable cell migration and proliferation in a choreographed manner, a significant proportion of these biomolecule materials being present in their functional chemical structure, and is optionally resorbable. The sol-gel-based formulation is at the heart of a biosensor platform. Sol-gels are also known to be useful for providing medical devices with immobilized and/or releasable biomolecules.

All of the disclosures contained herein focus on the issues of maintaining active biomolecule material within the formulations and optionally releasing the biomolecule material in its functional chemical structure.

### BRIEF DESCRIPTION OF THE SEVERAL VIEWS OF THE DRAWINGS

Figure 1 is a simplified schematic of the formulation production and screening system.
Figure 2 is a graphical representation of the shift in emission maxima which occurs upon a change of conformation of a biomolecule.
Figure 3 is a schematic representation of the screening process of the disclosure.
Figure 4 is a schematic representation of exemplary procedures used to determine the total amount of protein released from a screened film and the fraction of total protein that is achieved upon release from the film.
Figure 5 is a representation of an exemplary medical device prepared with a composition according to the disclosure herein.
Figures 6-8 are cross sectional views of coated medical devices.
Figure 9A is a graphical representation of the shift in emission maxima of a biomolecule with increasing concentration of chemical denaturant.
Figure 9B is a graphical representation of the shift in emission maxima of a biomolecule which occurs upon storage.
Figure 10 is a graphical representation of the results obtained in Example 8.
Figure 11 is a graphical representation of the release characteristics of a laminate composition containing different biomolecule in each layer of the laminate.
Figure 12 is a graphical representation of the biomolecule release characteristics from a film of constant thickness with varying average molecular weight of the biodegradable polymer.

### DETAILED DESCRIPTION

The formulations and medical devices prepared with formulations identified by the screening methods of the present disclosure may be utilized to effectively prevent and treat such medical conditions as, for example, large conductive airway stenosis and, in particular, large conductive airway stenosis caused by injury. They are also of use in biosensor development.

While exemplary embodiments of the disclosure will be described with respect to the treatment of stenosis and/or restenosis and related complications of injury of a large conductive airway, it is important to note that the local delivery of biomolecule formulations having a significant proportion of the biomolecule material in its functional chemical structure may be utilized to treat a wide variety of conditions utilizing any number of medical devices, or to enhance the function and/or life of the device.

The expression "significant proportion" as utilized herein refers to at least about 50% of the total biomolecule present in the formulation is present in its chemically active structure as determined by emission spectroscopy or enzyme linked immunosorbent assay.

The term "biomolecule" as utilized herein refers to a molecule of biological origin or synthetic analog thereof capable of promoting a desired cellular response in an organism receiving a therapeutic amount of the biomolecule, or capable of serving as a biosensor. The term biomolecule is intended to encompass molecules or groups of molecules forming functional chemical structures and having a detectable deformation of the functional chemical structure, the deformation serving to render the materials inactive. Therefore, such biomolecule materials would include, as non-limiting and non-exclusive examples: peptides, polypeptides and proteins.

The expression "functional chemical structure" as utilized herein refers to that structure or conformation of the biomolecule that provides the specificity and/or reactivity of the biomolecule. A "functional chemical structure" as utilized herein is detectable by analytical methods. Also, a deformation of the functional chemical structure which serves to render the biomaterial inactive is detectable by analytical methods.

The term "peptide", as used herein, refers to a compound consisting of from two to about ninety amino acid residues wherein the amino group of one amino acid is linked to the carboxyl group of another amino acid by a peptide bond. A peptide can be, for example, derived or removed from a native protein by enzymatic or chemical cleavage, or can be prepared using conventional peptide synthesis techniques (e.g., solid phase synthesis) or molecular biology techniques (see, for example, Sambrook, J. et al., Molecular Cloning: A Laboratory Manual, Cold Spring Harbor Press, Cold Spring Harbor, N.Y. (1989)). A "peptide" can comprise any suitable L- and/or D-amino acid, for example, common α-amino acids (e.g., alanine, glycine, valine), non-α-amino acids (e.g., β-alanine, 4-aminobutyric acid, 6-aminocaproic acid, sarcosine, statine), and unusual amino acids (e.g., citrulline, homocitrulline, homoserine, norleucine, norvaline, ornithine). The amino, carboxyl and/or other functional groups on a peptide can be free (e.g., unmodified) or protected with a suitable protecting group. Suitable protecting groups for amino and carboxyl groups, and methods for adding or removing protecting groups are known in the art and are disclosed in, for example, Green and Wuts, "Protecting Groups in Organic Synthesis", John Wiley and Sons, (1991). The functional groups of a peptide can also be derivatized (e.g., alkylated) using art-known methods.

The term "protein" as utilized herein refers to any of a large group of organic molecules found as major macromolecular constituents of living organisms. Therefore the term "protein" would include all types of polypeptides, enzymes, antibodies, etc. and is intended to be a broadly inclusive term as utilized herein and would therefore include multi-subunit proteins, conjugated proteins, as well as protein complexes.

The antibody can be polyclonal or monoclonal, and the term "antibody" is intended to encompass both polyclonal and monoclonal antibodies. The terms polyclonal and monoclonal refer to the degree of homogeneity of an antibody preparation, and are not intended to be limited to particular methods of production. The term "antibody" as used herein also encompasses functional fragments of antibodies, including fragments of human, chimeric, humanized, primatized, veneered or single chain antibodies. Functional fragments include, but are not limited to, Fv, Fab, Fab' and F(ab')₂ fragments. Such fragments can be produced by enzymatic cleavage or by recombinant techniques. For example, papain or pepsin cleavage can generate Fab or F(ab')₂ fragments, respectively. Other proteases with the requisite substrate specificity can also be used to generate Fab or F(ab')₂ fragments. A more thorough discussion of antibodies can be found in, for example, U.S. Patent Appl. Ser. No. 09/809,739, published August 8, 2002, as publication number 20020106369.

The term "peptidomimetic", as used herein, refers to molecules which are not polypeptides, but which mimic aspects of their structures. For example, polysaccharides can be prepared that have the same functional groups as peptides. Peptidomimetics can be designed, for example, by establishing the functional chemical structure of a peptide agent in the environment in which it is bound or will bind to a target molecule (e.g., cellular adhesion molecule, chemokine receptor). The peptidomimetic comprises at least two components, the binding moiety or moieties and the backbone or supporting structure. A peptidomimetic useful in the practice of the invention disclosed herein will have a detectable emission spectra. A more thorough discussion of peptidomimetics can be found in, for example, U.S. Patent Appl. Serial No. 09/809,739, published August 8, 2002, as publication number 20020106369.

Methods of screening formulations according to the disclosure herein involve printing a small volume of formulation onto a substrate. Methods of printing are well known to those of ordinary skill in the art. A description of suitable printing methods may be found in Mark Schena, ed., Microarray Biochip Technology, Bio Techniques Press, Westborough, Massachusetts (2000). Methods of printing would include pin printing, inkjet type printing, etcetera.

Pin printing involves direct contact between the printing mechanism and the substrate. Although pin printing may be performed manually, to obtain improved results, use is frequently made of electro-mechanical pin printing devices such as the ProSys 5510 System available from Cartesian Technologies, Inc. of Irving, California. Ink jet printing is characterized by through space ejection of the printing formulation and is considered to be a form of non-contact printing.

In pin printing, pin tools are dipped into the screening formulation or loaded with same resulting in the transfer/dispersion of a small volume of fluid onto and/or within the tip of the pins. Pin tools deliver sample spots of the screening formulation onto the substrate and include solid pins, capillary tubes, tweezers, split pins and micro-spotting pins or "ink stamps". Touching the pins or pin samples onto the substrate leaves a spot, the diameter of which is determined by the surface energies of the pin, fluid, and substrate; and the pin velocity. The pins typically have a loading volume of about 0.2 to about 0.6 µL and can produce spots ranging from about 60 to about 400 µm in diameter, depending on printing solution surface properties.

The screening method in general begins with the preparation of a series of formulations under ambient conditions. Reagents used in preparing formulations would include biomolecules, solvents, protectant material such as surfactant capable of forming reverse micelles or ionomer capable of forming protective domains, and matrix material.

Biomolecules suitable for use in preparing formulations would include those materials set forth above in the definition of "biomolecule." Therefore, biomolecules would include enzymes, peptides, polypeptides, proteins, antibodies, peptidomimetics, drugs, etcetera.

Solvents suitable in preparing formulations would include any solvent into which the polymer, when a polymeric matrix is used, is soluble to at least 0.1 wt% (e.g., methylene chloride, chloroform, *n*-octane, *iso*-octane, *n*-heptane, etc.). Other solvents useful in preparing the formulations for screening would include those known in the art to be useful in preparing sol-gels, bioceramics, and bioglasses (e.g., water, alcohol). As utilized herein the term solvent is intended to be inclusive of liquids used as carrier mediums for dispersions such as may be used in the preparation of sol-gels, bioceramics or bioglasses.

A protectant material such as a surfactant which is known to form reverse micelles or an ionomer which is known to form protective domains may be used within the formulation to aid in maintaining a biomolecule material contained in the formulation in its functional chemical structure. In this context, we have discovered that surfactants which are known to form reverse micelles in a particular formulation increase the percentage of biomolecule material which is in its functional chemical structure. Many surfactants may form reverse micelles. The issue depends on the solvent used, the water loading, the molar ratio of surfactant to water, and the temperature. Although not intending to be bound by any theory, it is considered that the protection of the biomolecule is being provided by the formation of reverse micelles. The surfactants may play a role to stabilize the protein surface within the formulation by forming protective domains other than reverse micelles. Other materials, such as ionomers, which are known to form protective domains in formulations may be used in the formulation to aid in maintaining the biomolecule material in its functional chemical structure.

Methods of forming reverse micelles in formulations are known to those of ordinary skill in the art. (See, for example, U.S. Appl. Ser. No. 10/099135, published March 20, 2003, as publication number 20030054036. Based upon the method of preparing and screening formulations disclosed herein, it would be a matter of routine experimentation to identify any particular protectant material useful in maintaining the biomolecule in its functional chemical structure based upon the particular components of the formulation. Ionic surfactants, including cationic, anionic and zwitterionic surfactants, are suitable hydrophilic surfactants for use in the present disclosure. Examples of ionic surfactants include fatty acid salts, bile salts, carnitines, etcetera. Specific examples of ionic surfactants would include sodium oleate, sodium lauryl sulfate, sodium lauryl sarcosinate, sodium dioctyl sulfosuccinate, sodium cholate, sodium taurocholate, lauroyl carnitine, palmitoyl carnitine, and myristoyl carnitine. Other surfactants are known to those skilled in the art. Examples of surfactants which may be suitable depending upon the formulation can be found in, for example, U.S. Patent No. 6,309,663 issued October 30, 2001. Preferred surfactant and concentration of the surfactant in the formulation will be identifiable to those of ordinary skill in the art through use of the screening method disclosed herein. Ionomers demonstrating microphase domain separation morphology such as the pefluorinated ionomer Nafion^{®} (perfluorosulfonate ionomer), as well as the copolymer of ethylene and methacrylic acid Surlyn^{®}, may also increase the percentage of biomolecule material present in the formulation in its functional chemical structure. Because ionomers are continuing to be developed, any ionomeric material having a small number (e.g., less than 10 mole percent) of the polymer repeat units having ionic functionality covalently bonded to the polymer backbone that have been developed or will be developed would be expected to demonstrate sufficient microphase domain separation morphology sufficient to provide a significant proportion of the biomolecules having active chemical structure.

To form a biosensor composition or implantable medical device, a holding material is preferably used as the matrix in the screening formulation. Any liquid material known to those of ordinary skill in the art for holding, immobilizing, entrapping, and/or sequestering biomolecules, can be used for screening according to the method disclosed herein. Suitable holding materials include, but are not limited to, sol-gels, xerogels, aerogels, protein-doped xerogels, organically-modified silanes, acrylamide gels, organic polymers, inorganic polymers, and mixtures thereof. One commonly used holding material is a sol-gel-derived glass. A sol-gel-derived glass is a porous glass formed by the condensation and polycondensation of one or more metal or semi-metal alkoxide mixtures with water and an acid catalyst. Sol-gel-derived glasses provide a convenient means to sequester biomolecules, because they can prevent undesirable leaching from the holding material, and the glasses themselves are porous, thereby allowing analytes to penetrate into the glass, and react with the chemical sensors or allowing bodily fluids, such as interstitial fluid, to contact the biomolecule contained within a medical device comprising sol-gel-derived materials. Sol-gel processed xerogels are also useful for holding protein based chemical sensors. It is known that protein-doped xerogels demonstrate k_{cat}, kₘ or k_{binding} for biomolecules within the xerogels that are substantially unchanged from the values in solution and the xerogel-doped biomolecules remain stable for relatively long periods of time. Although these demonstrated k values might otherwise be viewed as teaching away from the need to use the screening method of the present disclosure for xerogel applications, it turns out that the optimization of a given formulation to yield an active biomolecule is still somewhat of an art. A trial and error, Edisonian approach has been used in the past, but that is very laborious. The screening method of the present disclosure helps to speed things up by at least 100-fold and allows one to zero in on a good/excellent formulation fast. This, of course, allows one to then determine the features of the "best" formulations and mimic them for other systems. Methods of making sol-gel-derived materials are known (see, e.g., U.S. Patent No. 5,874,109, issued February 23, 1999).

Therefore, the type of matrix material that may be used in the formulations, devices and methods of this disclosure is virtually limitless and may include both biological and synthetic matrices. The matrix material intended for use in medical devices will have all the features commonly associated with being "biocompatible" in that it is in a form that does not produce an adverse, allergic or other untoward reaction when administered to a mammalian host. Such matrices may be formed from either natural or synthetic materials, or both. The matrices may be non-biodegradable in instances where it is desirable to leave permanent structures in the body; or biodegradable where the desired tissue response is required only for a relatively short duration of time. For example, the formulations may be used to form medical devices such as sponges, laminates, implants, tubes, telfa pads, band-aids, bandages, pads, lyophilized components, gels, patches, powders or nanoparticles. In addition, formulations can be designed to allow for use as biosensor compositions in which case biocompatibility may not be of particular concern.

The choice of matrix material will differ according to the particular circumstance and the site that is to be treated. Physical and chemical characteristics, such as, e.g., biocompatibility, biodegradability, strength, rigidity, interface properties and even cosmetic appearance may be considered in choosing a matrix material, as is well known to those of ordinary skill in the art. Appropriate matrices may both deliver the biomolecule and also act as an in-situ scaffolding through which a desired tissue response may occur.

In certain embodiments, biodegradable or resorbable matrices may be employed. A biodegradable matrix is generally defined as one that is capable of being resorbed or remodeled into the body. Potential biodegradable matrices for use in connection with the compositions, devices and methods of this disclosure include, for example, sol-gel-derived materials including bioceramics such as biodegradable and chemically defined calcium sulfate, tricalciumphosphate, hydroxyapatite, sintered hydroxyapatite; bioglass, aluminates, xerogels, organically-modified silanes, other bioceramic materials or bioglass materials, polymers such as polylactic acid and polyanhydrides; matrices of purified proteins, and semi-purified extracellular matrix proteins. Nonbiodegradable biocompatible polymers such as, for example, styrene and acrolein, may also be useful in the practice of the disclosure herein.

Non-limiting examples of polymers useful in the practice of the disclosure herein include polyesters, polyanhydrides, polyolefins, polyvinyls, polymethacrylates, polyalkylcyanoacrylates, polyacrylamides, polyorthoesters, polylactones, polycaprolactones, polyphosphazenes, polypeptides, polystyrenes, polyethylenes, polyethers, polyamides, polycarbonates, polyalkylenes, polyurethanes, copolymers thereof and mixtures thereof.

When the polymer component is a polyester, the polyester may be formed of poly(α-hydroxy acids), poly(β-hydroxy acids), poly(α-malic acids), pseudo poly(α-amino acids), copolymers thereof and mixtures thereof. Examples ofbioabsorbable aliphatic polyesters (i.e. homopolymers and copolymers of lactic acid, glycolic acid, lactide, glycolide, para-dioxanone, trimethylene carbonate, ε-caprolactone, and blends thereof). When the polymer component is a polyanhydride, the polyanhydride may be formed of homo-polyanhydrides of sebacic acid, homo-polyanhydrides of fumaric acid, random co-polyanhydrides of sebacic and fumaric acids, and mixtures thereof.

It is to be understood that virtually any polymer that is now known or that will be later developed and that may be suitable for the sustained or controlled release of biomolecules, as well as such sol-gel-derived materials as may be suitable as a holding agent for a biomolecule useful in sensor applications or as an implantable device may be employed in the present disclosure.

Generally, the selection of a particular polymer or copolymer depends critically upon the specific biomolecule in question and issues like the biomolecule's surface charge, isoelectric point, form (monomer vs. n-mer), etcetera. However, through the use of the screening method disclosed herein, the selection of particular polymers and/or copolymers will not be critical because the high throughput screening method will identify those formulations that are best suited to a desired application and/or specific biomolecule.

A wide range of biomolecule loadings can be used in connection with any of the above matrix materials to provide a formulation. The amount of loading being readily determined by those of ordinary skill in the art and ultimately depending upon the desired application of the formulation or the condition to be treated, the nature of the biomolecule itself, the means by which the biomolecule-loaded formulation is administered to the intended subject, and so forth. The loaded formulation will typically comprise from about 1 to about 1000 ppm biomolecule.

The formulation is used to provide the entirety of a sensor or medical device or a portion of a medical device. Portions of medical devices for which the formulations find use include any fraction of a medical device, such as device coatings, laminates, device components and so forth. While not a requirement of the present disclosure, the materials, coatings, and films identified by the screening process disclosed herein may be provided as laminates. For example, to improve the physical properties of the medical device and/or to provide for the choreographed release ofbiomolecule(s) and desirable cofactors, cell nutrients, etc., the coatings can be applied as laminates to or formed as laminated medical devices. When desirable, crosslinking may be effected by any of the known crosslinking mechanisms, such as chemical, heat or light. In addition, crosslinking initiators and promoters may be used where applicable and appropriate. In those exemplary embodiments utilizing crosslinked films comprising biomolecules, curing may affect the rate at which the biomolecule is released from the coating. Crosslinked copolymers films and coatings may be used without biomolecule material to modify the surface of implantable medical devices. The crosslinking may take place throughout a particular layer or layers of a device, or may only occur at the interface of the layer or layers of a device prepared according to the disclosure herein.

In some instances, biomolecule is released from the device or device portion to a bodily tissue or bodily fluid upon contacting the same. An extended period of release (i.e., 50% release or less over a period of 24 hours) may be preferred in some cases. In other instances, for example, in sensor formulations or in cases where enzymes, peptidomimetics and other agents capable of acting on a substrate are used as the biomolecule, the biomolecule may remain within the formulation matrix. It is also possible to prepare formulations or medical devices comprising formulations wherein biomolecules are released in a choreographed manner.

It is known that many growth factors, secondary messengers, nutrients, and other cellular signaling molecules can be involved in generating a biological response. For example, keratinocyte growth factor (KGF) plays an important role in wound repair by increasing epithelial cell migration and proliferation. It has been shown that the respiratory epithelium initiates an autocrine intra-epithelial repair process in response to injury by expressing proteins that have known mitogenic and motogenic activity (e.g., KGF; transforming growth factor alpha (TGF-α)). Given this, we have developed biodegradable polymer-based formulations that can release active KGF and, optionally, other cytokines/growth factors in a choreographed manner to improve wound healing within, for example, the human LCA. These formulations can be tailored for a variety of biomedical applications such as, for example, anastomosing devices, stents, sutures, etcetera.

The choreographed release ofbiomolecules from a medical device is attained through the use of a multi-layer medical device. The choreographed delivery device comprises a laminate having at least two layers, each of the two layers containing a distinct biomolecule. The laminate is configured in such a manner that primarily a single layer is exposed to the degradation conditions. The layers are selected by the screening method disclosed herein to provide a significant proportion of the biomolecule released in its active form. The formulation is also selected for its decay kinetics. The underlying layer does not undergo significant degradation until the outermost layer is substantially degraded. At this time the underlying layer can begin the degradation process. Because of the selection of formulations and providing the formulations as a laminate, choreographed delivery of biomolecules is provided.

As a non-limiting example of biomedical applications, disclosed herein are devices for maintaining integrity of the large conducting airway. It will be understood by those of ordinary skill in the art that devices could be formed of the materials disclosed herein for use in any lumen of the body as well as other locations in a body. Also, the materials disclosed herein are useful in any mammal and not just primates.

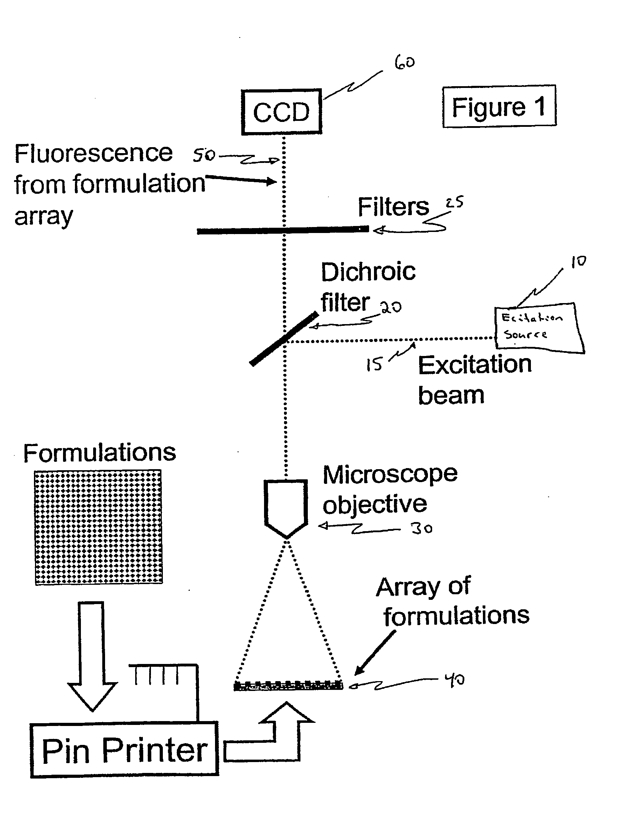

The method of screening disclosed herein will now be discussed in reference to certain of the figures, in which Figure 1 is a schematic diagram of an apparatus useful to prepare and screen arrays of formulations. The excitation source 10 is directed to an epi-fluorescence microscope (only certain elements of which are shown). The excitation beam 15 is reflected off the front face of a dichroic filter 20 to a microscope objective 30 that serves to illuminate the array of formulations 40. The resulting luminescence from the array is collected and passed through the dichroic filter 20 and any residual excitation radiation is suppressed further by an optical filter 25. Any number of narrow bandpass interference filters 25 may be used to study the emission spectra from within each formulation. In protein systems, the main emission arises from the amino acids tryptophan, tyrosine, and phenylalanine (other, non-natural amino acids also fluoresce). Any natural luminorphores, e.g., NAD, NADH, that are associated with the protein may also emit radiation upon excitation.

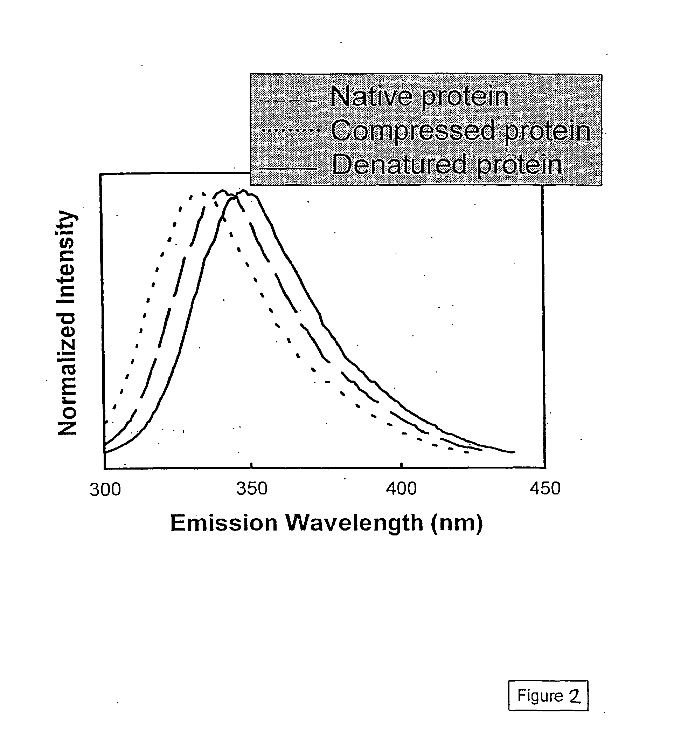

Figure 2 presents typical emission spectra for native (-----), compressed (.....), and denatured (____) human serum albumin. The emission spectra provide a direct measure of the changes in the protein structure. That is, if a spectrum deviates from native, the protein is not, by definition, in its native conformation. The focus of whether an emission spectra deviates from the native spectra is based upon a comparison of the maximum and the full width at half maximum of the formulation emission spectra and the native biomolecule emission spectra. Thus, a formulation spectra that is closest to the native spectra will be that spectra that has the most similar maximum and the full width at half maximum as the native biomolecule emission spectra (the control emission spectra). We use the intrinsic protein tryptophan emission spectrum as the basis to rapidly screen (using the apparatus shown in Figure 1) the protein-loaded, formulations to identify only those formulations that yield protein tryptophan emission spectra that are the most like the native protein in buffer. Depending upon the particular biomolecule, the emission spectra of other molecules in the biomolecule could be used to monitor the functional chemical structure of the biomolecule. The native conformation, for biomolecules which demonstrate reaction and/or binding specificity, is the conformation that provides the functional chemical structure of the biomolecule. The luminescence 50 from the array of formulations 40 is suitably imaged, for example, on to the face of a thermoelectrically-cooled charge coupled device 60. The detected images are acquired, displayed, and processed by using suitable software. Typically, the images are acquired as a false color CCD image of a portion of an array of formulations containing monoclonal anti-fluorescein antibodies after the array was subjected to fluorescein and rinsed to displace non-specifically adsorbed fluorescein. Each spot is a different formulation. The intensity from each spot is a measure of the anti-fluorescein activity within each formulation. The brighter the spot, the greater the amount of the biomolecule that is present in its chemically active structure.

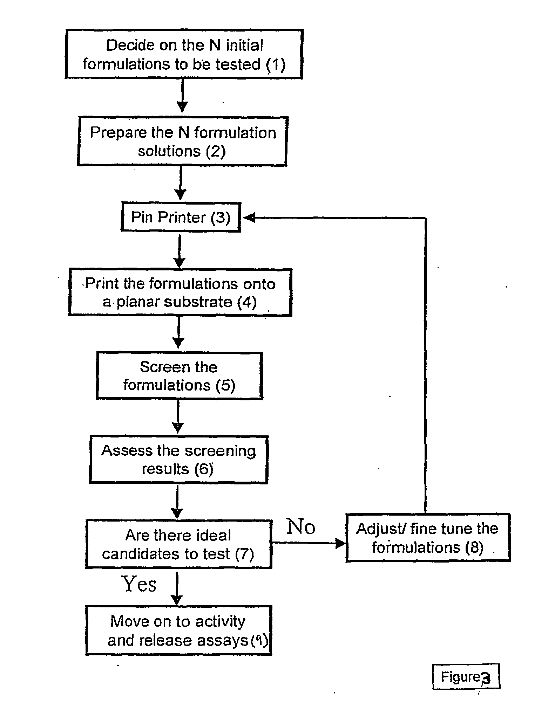

Figure 3 is a schematic representation of the steps employed in the method of screening disclosed herein. Step 1 involves deciding on the number of initial formulations to be screened. The next step (Step 2) involves the preparation of the various formulations to be screened. Preparation of the formulations includes preparing mixtures or solutions of the biomolecule(s), solvent(s), protectant material, and matrix material(s) and loading the different formulations into suitable containers, for example, the various wells of a 96 well plate. Step 3 involves loading the printing device, for example, a pin-printer, with the formulations to be tested. Once the printing device is loaded, the formulations are printed onto a planar substrate (Step 4).

Formulations can be printed onto, for example, fused silica or glass microscope slides. The liquid formulations are printed directly onto the clean microscope slides by using, for example, a ProSys 5510 system (Cartesian Technologies; Irvine, California) with model CMP-3 pins (TeleChem; Sunnyvale, California). The print chamber relative humidity is maintained at conditions appropriate to the formulations being screened, for example, between 30 and 40%. The final dimensions of each formulation "spot" is a function of the formulation composition, relative humidity, pin contact time with the substrate, and substrate's surface chemistry. The individual spots of formulations are about 100-150 µm in diameter and they are generally reproducible to about 10% RSD. Scanning electron microscopy shows the spots of formulations are typically about 1-2 µm thick, depending on the exact formulation printed, the pin-to-substrate contact time, and the substrate's surface chemistry. The time required to print each formulation is ∼ 1 s. Pins are washed between each formulation, for example, by using the ProSys' cleaning program.

Formulations are printed and screened to identify those which exhibit emission spectra that are equivalent to the spectrum in buffer and, optionally, exhibit other desirable properties such as emission spectra that do not shift during one month of refrigerated storage. If the formulation exhibits an emission spectrum that is between that of native and fully chemically denatured biomolecule, it is not likely to release active biomolecule. A formulation yielding an emission spectrum that is indistinguishable from native biomolecule in buffer is more likely to release active biomolecule. Therefore formulations are selected as leads on the basis of which formulations display emission spectra closest to the control emission spectra.

All formulations are prepared, printed, and screened by using high-speed printing techniques in concert with an appropriate detector device. By using these apparatuses one can prepare and screen about 600-700 formulations per hour. It will be understood by those of ordinary skill in the art that experiments should be performed on separate occasions using separate reagent batches to ensure the accuracy of the results. Blanks for each formulation should also be prepared and printed. It is desirable that all arrays of formulations be allowed to age in the dark for a suitable period of time prior to screening.

Step 5 involves the screening of the formulations. After the entire array is prepared, emission data is recorded and screened. The screening is intended to identify emission spectra that are indistinguishable from biomolecule dissolved in buffer, both fresh and preferably after proper storage. The screening also identifies emission spectra between native and fully denatured biomolecule. A formulation exhibiting the emission spectra of a fully denatured biomolecule would not be expected to deliver a significant percentage of active biomolecule upon film biodegradation. Formulations are prepared and screened until a formulation which is indistinguishable from native biomolecule dissolved in buffer is identified as a promising candidate to release active biomolecule.

The doped formulations are screened at two levels. Initially, the intrinsic emission spectra from within each doped formulation is recorded. Because tryptophan fluorescence is sensitive to the local microenvironment that surrounds the residue in a protein, its emission spectra is useful for studying the structural relationship of a biomolecule to various formulations. Other residues that exhibit similarly desirable monitoring properties may also be selected for studying the structural relationship of a biomolecule to various formulations. Thus, for example, changes in the KGF emission spectrum, in comparison to the native KGF spectrum, provides a rudimentary measure of the KGF structure within the formulations. The full description of these spectroscopic measurements has been discussed in, for example, Cho, EJ, et al., Appl Spectrosc.; 56:1385-1389 (2002).

The formulations are assessed as follows: (A) The entire array is immersed in buffer. (B) The detected response for the array of formulations is recorded within a suitable period of time to avoid significant (< 1%) biodegradation or photobleaching (< 3%). (C) From the intensity at each emission wavelength for each formulation is constructed a biomolecule emission spectrum by fitting the data points to a log normal function. A suitable intrinsic residue emission spectra from the formulations is selected to assess the gross structure of the biomolecule within the formulations prior to liberation. Examples of suitable residues would include tryptophan, tyrosine, phenylalanine, plus any natural luminorphores (NAD, NADH) within proteins or unnatural fluorescent amino acids. A suitable intrinsic residue emission spectra would be one that provides a detectable shift as the functional chemical structure is deformed, as in chemical denaturation of the biomolecule in buffer solution. To rapidly assess any structural changes in the sequestered biomolecule, the emission spectra from within each formulation is recorded. Some emission spectra such as tyrosine and tryptophan emission spectra are sensitive to the local microenvironment that surrounds these residues within any biomolecule such as a protein. Therefore, changes in the physicochemical properties surrounding the biomolecule can alter the biomolecule's emission spectra. The emission maximum of the biomolecule in buffer as a function of added denaturant can be monitored. As the denaturant concentration increases, the emission spectrum shifts because the biomolecule denatures and the average tryptophan residues moves from a hydrophobic to a hydrophilic microenvironment. (Note: This shift may not be seen for all emission spectra.) If the biomolecule maintained its functional chemical structure and was not altered by the film host, the emission maximum would remain constant at the native emission maxima.

Once those formulations that exhibit emission spectra that are the most like native spectra are identified, casting methods can be used to prepare test films of these "lead formulations for further study. These films are loaded with labeled biomolecule and used to determine the release kinetics from the lead formulations and quantify the fraction of active biomolecule released from the lead formulations.

Standard immunoassay testing, such as enzyme linked immunosorbent assay, can be performed upon the biomolecule to determine whether it is in its active chemical structure both within the formulation as well as released from the formulation. The immunoassay should be sensitive to slight changes in the conformation of the biomolecule. Antibodies that readily recognize native biomolecules and selectively bind only to the native biomolecules in a binary mixture that contains active and denatured biomolecule are desirable. Such an assay allows one to effectively discriminate, in a rigorous fashion, between active and inactive forms of biomolecule as well as determining the concentration of active biomolecule that is liberated from the polymer-based formulations.

To assess the screening results (Step 6), the biomolecule emission maximum is compared for each formulation to biomolecule emission maximum in non-denaturing buffer solution to identify those formulations that are the most likely to release active biomolecule from the host matrix or contain immobilized biomolecule in its active chemical structure. That is, those formulations that yielded biomolecule emission spectra that are the most similar to biomolecule dissolved in buffer are considered to be the best performing/most viable. After assessing the results in Step 6, a decision is made in Step 7 as to whether there are any lead candidates identified as possible formulations for further study. If no leads are identified, then Step 8 involves adjusting or otherwise fine tuning the formulations and repeating Steps 3 through 7. If a lead candidate or candidates is identified then Step 9 involves performing activity and release assay of the lead candidate(s). If desired, bioassays on the biomolecule that is released (Step 9) can be performed from several formulations to determine the percentage of the total biomolecule that is released that is able to bind to suitable antibodies. This is deemed to be the percentage of active biomolecule from a given formulation.

Knowing this, one can apply our method to prepare, screen, and identify formulations that do not alter the emission spectrum (i.e., biomolecule functional chemical structure) with the idea being that these formulations would in turn be the most capable of delivering active biomolecule as they biodegraded. Toward this end, a statistical design strategy is used to prepare a series of blended formulations based on various components of the formulation as well as pH and casting solvents. Surfactants capable of forming reverse micelles or ionomers capable of forming protective domains can be used in an effort to form an environment into which the biomolecule would reside and remain stable within the formulation. There is a body of literature on the use of reverse micelles to stabilize a wide variety of proteins in organic solvents and on the use of surfactants to form drug/protein loaded biodegradable formulations and/or to control formulation biodegradation kinetics.

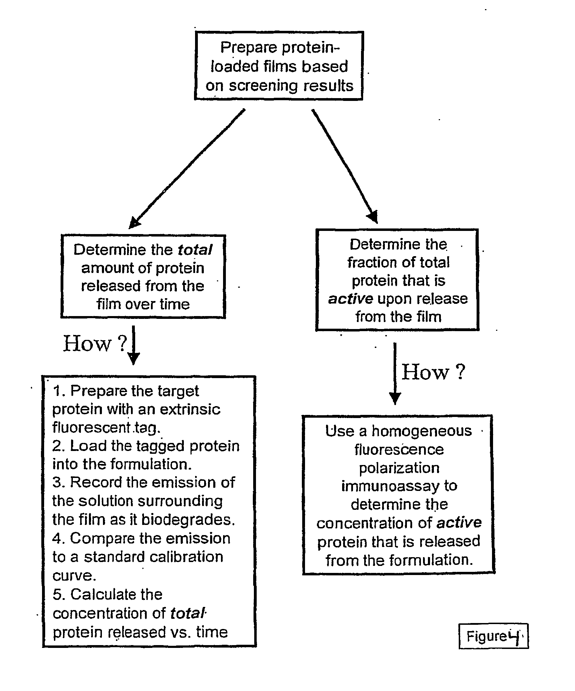

As shown in Figure 4, in certain embodiments of the disclosure herein, the analysis (Step 9) of the biomolecule release characteristics of the screened formulations is begun by preparing lead specimens based upon the screening results and mounting them on/in suitable sample holders. An example of a suitable specimen is a thin film specimen. Methods of preparing thin film specimens of the formulations include, for example, melt pressing, melt casting, spin coating, solvent casting, monolayer film production, lamination, etc. When desirable, such as for monitoring the release characteristics of a screened formulation, the thin film specimen can be placed in a specimen holder which can hold a degrading medium and be subjected to further analysis, for example, a fused quartz cuvette. Other suitable sample holders will be apparent to those of ordinary skill in the art.

Degradation of the lead specimen can be performed by exposing the material to lytic solution, acids, bases, etc., abrading the specimen, solvent immersion, buffer solution immersion. The degradation can take place at any suitable temperature, for example, room temperature, body temperature, etc. When degradation is accomplished by hydrolyzing in buffer solution, the buffer solution can be various saline buffers having a pH between about 2.0 and about 12.0 and containing ions such as phosphate, acetate, carbonate, biphthalate, or mixtures thereof.

Samples of the degrading medium can be taken to determine the release profile (such as by HPLC, UV analysis or use of radiotagged molecules). The determination of the total amount ofbiomolecule (in the case of Figure 4, protein) released from the film over time is accomplished through a multi-step process. The target biomolecule is labeled with an extrinsic tag, for example, a fluorescent tag. The tagged biomolecule is loaded into the formulation and the signal from the tagged biomolecule is recorded from the solution surrounding the loaded film as the film degrades. This signal is then compared to a standard calibration curve. The comparison of the signal obtained from the loaded film over time as the film degrades to the standard curve allows for the calculation of total biomolecule released as a function of time. To determine that fraction of the total biomolecule released that is in its functional chemical structure, a concurrent assay such as a homogenous fluorescence polarization immunoassay can be performed. Such an assay is sensitive only to biomolecule that is present in its functional chemical structure.

The total biomolecule released from a formulation during degradation can be tracked by loading the lead formulations with labeled biomolecule to determine the total biomolecule concentration released vs. time profile. Film samples of the labeled formulations are placed in a standard cuvette containing a suitable degradation media. The analytical instrument, for example, a spectrofluorimeter, is configured to excite the labeled biomolecule and collect its emission in the solution surrounding the film. The film itself is not excited during these experiments; only signal from the buffer, arising from liberated labeled biomolecule is measured. As the films degrade, the labeled biomolecule is liberated into the buffer and the total biomolecule concentration over time is determined from the observed signal intensity and a series of standard calibration curves.

The fraction of active biomolecule released from the formulations can also be determined. As utilized herein, "active" biomolecule refers to the species that has the same epitopes of interest as the native biomolecule. For example, an active biomolecule may be the species that binds to the anti-biomolecule antibody. To determine the percentage of active biomolecule released from the lead biodegradable polymer formulations ([active/total] x 100%), we use, for example, a steady-state fluorescence anisotropy immunoassay. Other immunoassays may also be used such as enzyme-linked immunosorbent assays and the like. Standard titrations on a series of biomolecule samples is performed initially to confirm the ability to simultaneously discriminate and quantify active and inactive biomolecule. To this end preliminary test samples would include: [1] Native biomolecule dissolved in buffered saline that had not been subjected to any chemical denaturant. This sample represents active biomolecule. [2] Biomolecule that has been subjected to mild denaturing conditions. This sample should also represent "active" biomolecule. [3] A mixture of biomolecule that was subjected to strongly denaturing conditions plus an equal molar amount of native, non-denatured biomolecule. This sample represents a mixture of active and inactive biomolecule. The denaturant is removed from samples [2] and [3] by dialysis to minimize inadvertent anti-biomolecule antibody denaturation that might bias the assay results.

The release kinetics and activity upon release from formulations can also be determined by the method of this disclosure. Kinetic and activity results for biomolecule is obtained from formulations that are screened. Screening according to the method of this disclosure serves to identify whether a strategy adopted as a universal approach for delivering active proteins from within a biodegradable polymer formulation actually works.

The screening method of this disclosure also provides for tuning of the release kinetics of the biomolecule. As discussed above, the release kinetics and activity upon release from formulations can be determined. Therefore, formulations can be prepared and selected based upon desirable release properties. An ideal biodegradable device would provide a means to tune the release kinetics to control the delivery rates. The release kinetics of a device can be tuned by using different concentrations of components within the formulations. Tuning the delivery from a formulation, for example, can be achieved by adjusting one property of one component within the formulation and simultaneously maintaining the activity. The formulations can be obtained by focusing and systematically varying aspects of the formulation such as molecular weight of the monomer (s) which form a polymer matrix, surfactant use, solvent used, as well as the concentration of the constituents of the formulation. The screening method will identify those factors that have an effect on the release kinetics as well as those factors that have no effect on release kinetics.

The use of a medical device prepared with a formulation identified by the screening method of this disclosure will now be discussed regarding treatment of stenosis within the human trachea/bronchus. Delays in reepithelialization within the human trachea/bronchus (large conducting airway - LCA) following traumatic injury, ablative surgical procedures, and/or congenital defects can lead to loss of lumenal integrity, causing airway compromise, with possible sepsis and death. It has been shown that the respiratory epithelium initiates an autocrine intra-epithelial repair process in response to injury by expressing proteins that have known mitogenic and motogenic activity (e.g., keratinocyte growth factor (KGF); transforming growth factor alpha (TGF-α)). This protein expression appears independent of connective tissue control. It has also been shown that interlukin-1 is produced by respiratory epithelial cells in culture during log phase growth and it too plays a role in cell migration and proliferation. Matrix metalloproteinases (MMPs), secreted by cartilage, appear to be a factor in the failure of complete LCA lumen reepithelialization after injury.

Therefore, implanting in an animal in need of treatment a medical device comprising a formulation having at least one biomolecule material contained therein, wherein the biomolecule material is selected to provide a desired cellular response to the injured tissue, and the biomolecule material is present in a therapeutically effective amount, wherein a significant proportion of the biomolecule material present in the medical device is present in its functional chemical structure, would be expected to provide a guided tissue response.

An example of a medical device for providing a guided tissue response is a stent either made of or coated with a polymeric composition prepared according to the disclosure herein. A stent is commonly used as a tubular structure left inside the lumen of a duct to relieve an obstruction. Commonly, stents are inserted into the lumen in a non-expanded form and are then expanded autonomously, or with the aid of a second device in situ. A typical method of expansion occurs through the use of a catheter-mounted angioplasty balloon which is inflated within the stenosed vessel or body passageway in order to shear and disrupt the obstructions associated with the wall components of the vessel or to otherwise maintain the desired architecture of the lumen being treated.

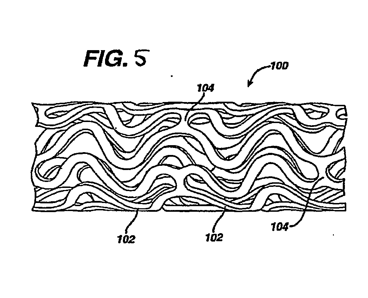

Figure 5 illustrates an exemplary stent which may be utilized in accordance with an exemplary embodiment of the present disclosure. The expandable cylindrical stent 100 comprises a fenestrated structure for placement in a blood vessel, duct or lumen to hold the vessel, duct or lumen open, more particularly for protecting a segment of LCA from stenosis after injury. The stent 100 may be expanded circumferentially and maintained in an expanded configuration that is circumferentially or radially rigid. The stent 100 is axially flexible and when flexed at a band, the stent 100 avoids any externally protruding component parts.

The stent 100 generally comprises first and second ends with an intermediate section therebetween. The stent 100 has a longitudinal axis and comprises a plurality of longitudinally disposed bands 102, wherein each band 102 defines a generally continuous wave along a line segment parallel to the longitudinal axis. A plurality of circumferentially arranged links 104 maintains the bands 102 in a substantially tubular structure. Essentially, each longitudinally disposed band 102 is connected at a plurality of periodic locations, by a short circumferentially arranged link 104 to an adjacent band 102. The wave associated with each of the bands 102 has approximately the same fundamental spatial frequency in the intermediate section, and the bands 102 are so disposed that the wave associated with them are generally aligned so as to be generally in phase with one another. As illustrated in the figure, each longitudinally arranged band 102 undulates through approximately two cycles before there is a link to an adjacent band 102.

The stent 100 may be fabricated utilizing any number of methods. For example, the stent 100 may be fabricated from a hollow or formed polymeric tube that may be machined using lasers, milling, chemical etching or other means. Alternatively the stent may be molded or extruded to have a desired shape. The stent 100 is inserted into the body and placed at the desired site in an unexpanded form. In one exemplary embodiment, expansion may be effected in a LCA by a balloon catheter, where the final diameter of the stent 100 is a function of the diameter of the balloon catheter used.

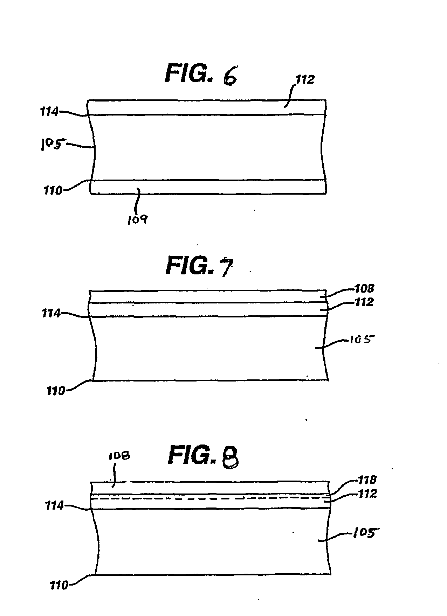

Figure 6 is a cross-sectional view of a medical device 105 having a coating containing biomolecule in its active chemical structure. The medical device 105 is coated on its outer surface 114 with a composition 112 comprising biomolecule in its active chemical structure. In the example of a stent for preventing stenosis of the LCA the composition 112 can consist of a PGA/PLA copolymer containing a surfactant capable of forming reverse micelles such as dioctyl sodium sulfosuccinate and KGF. This composition 112 has been identified as particularly useful for providing a significant proportion of KGF in its chemically active structure. Optionally, the medical device 105 can be coated on its inner surface 110 with a coating 109 that discourages the overgrowth of a medical device 105 with undesirable tissue or provides a coating 109 that improves the operation of the medical device with an insertion tool.

Figure 7 is a cross-sectional view of a medical device 105 having two separate coatings 112, 108 applied to its outer surface 114. The device 105 is prepared by first applying a coating 112 containing, for example, KGF. A second coating 108 containing, for example, interleukin-1(IL-1) is applied on top of the coating 112 containing KGF. The IL-1 coating 108 initially releases IL-1 as it degrades. Once the IL-1 coating 108 has degraded, the KGF containing coating 112 begins to release KGF as this coating 112 degrades. In this way the choreographed delivery of IL-1 and KGF is provided.

Figure 8 is the same as Figure 7 with the exception that the layers 108, 112 are crosslinked to each other through a crosslinking zone 118. The crosslinking zone 118 improves the adhesion of layer 108 to layer 112 and serves to decrease any undesirable premature delamination of the outermost layer 108 from its adjacent layer 112.

The invention will be better understood by way of the following examples which are presented for illustrative purposes and are not to be construed as limiting the claims in any way.

Unless stated otherwise, all regents were used as received. In the examples, aqueous solutions were prepared from deionized water that was treated with a Barnstead NANOpure II system to a specific resistivity of >18 MΩ cm; TRIS and phosphate buffers (pH 6-8, 1-100 mM) were prepared and used according to standard methods; recombinant human KGF (FGF-7) and KGF/FGF-7 antibody was obtained from R&D Systems (Minneapolis, Minnesota); IL-1, isotonic saline, and cellulose dialysis bags (12 kDa MW cut-off) were obtained from Sigma (St. Louis, Missouri); methylene chloride, chloroform, *n*-octane, *iso*-octane, and sodium bis(ethylhexyl)sulfosuccinate (Aerosol-OT, AOT), Dextran (25 000 MW), glycerol, and Nafion® (10 wt% dispersion in water) were obtained from Aldrich Chemical (St. Louis, Missouri); and *n*-heptane was obtained from Mallinckrodt (Hazelwood, Missouri). Sephadex G-25 disposable columns were obtained from Amersham Biosciences Corp. (Pisataway, New Jersey); BODIPY 650/665, BODIPY 630/650, Mouse monoclonal 4-4-20 anti-fluorescein antibodies, fluorescein, and fluorescein isothiocyanate (FITC) were obtained from Molecular Probes, Inc. (Eugene, Oregon); tetramethyorthosilane (TMOS) and aminopropyl triethoxysilane (APTES) were obtained from United Chemical Technologies (Bristol, Pennsylvania); and poly(lactic acid) (PLA, 2, 50, 100, 200, and 300 kDa) and poly(glycolic acid) (PGA), poly(ethyleneimine) (PEE, 70 000 MW, 30 wt% solution in water) and poly(ethyleneoxide) (PEO, 100 000 MW) were obtained from Polyscience, Inc. (Warrington, Pennsylvania).

A statistical design strategy was used to prepare a series of biodegradable polymer-based formulations (Tables 1 and 2 below).

Printing the Formulations for Screening. All formulations were prepared, printed, and screened by high-speed pin printer in concert with an epi-fluorescence microscope/charge coupled device detector. By using these apparatuses we can prepare and screen 600-700 formulations/hr.

All experiments were performed on five separate occasions using separate reagent batches. The average of all experiments is reported along with the corresponding standard deviations. Blanks for each formulation were also prepared and printed. All arrays of formulations were allowed to age in the dark at 4 °C for at least three days prior to screening.

Rapid Method to Assess Changes in Protein Structure within a Polymer-Based Structure. To test for any structural changes in the sequestered proteins, we recorded their tryptophan emission spectra. Tryptophan emission spectra are sensitive to the local microenvironment that surrounds this amino acid residue within a protein (cf., Figure 2). Therefore, changes in the physicochemical properties surrounding the protein alter the tryptophan residue spectra and this is used to assess the protein's structure and environment within a formulation.

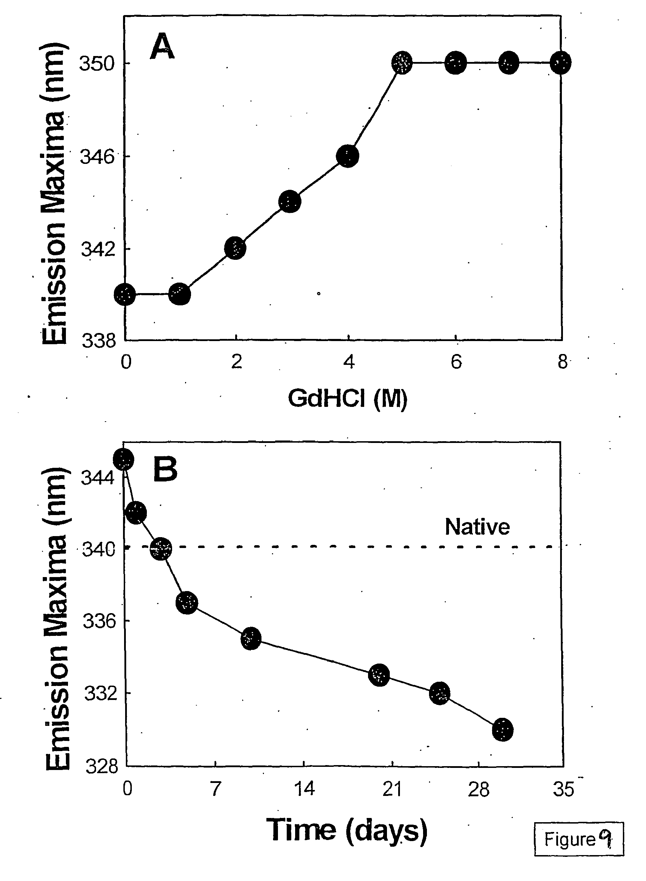

Figure 9A presents the KGF (Keratinocyte growth factor) tryptophan emission maximum in buffer (ex = 295 nm) as we add the chemical denaturant guanidine hydrochloride (GdHCl). As the concentration of GdHCl increases, the KGF tryptophan emission spectrum shifts to the red in line with the protein denaturing and the average tryptophan residues moving from a hydrophobic to a hydrophilic microenvironment. Figure 9B illustrates the effects of refrigerated storage time on the fluorescence from KGF sequestered within a pure PLA (polylactic acid) film cast from chloroform. If the protein conformation maintained its native structure in the PLA film and was not altered by the PLA film host, the emission maximum would remain at ∼340 nm (dashed line). This is not the case for this particular formulation. In fact, KGF expanded initially and then contracted as the KGF-doped PLA film aged. This behavior is not conducive to maintaining protein activity.

Basic Screening Strategy. The KGF-doped biodegradable polymer formulations were screened at two levels. Initially, we recorded the intrinsic KGF emission spectra (primarily from tryptophan residues) from within each KGF-doped formulation. Tryptophan fluorescence is sensitive to the local microenvironment that surrounds the residue in a protein. Thus, changes in the KGF emission spectrum, in comparison to the native KGF spectrum, provides a rudimentary measure of the KGF structure within the formulations. The full description of these spectroscopic measurements has been discussed in Cho, EJ, et al., Appl Spectrosc.; 56:1385-1389 (2002).

Once we identified those formulations that exhibited KGF emission spectra that were the most like native KGF, we used spin casting methods to prepare test films of these "lead" formulations for further study. These films were loaded with fluorescently-labeled KGF and they were used to determine the KGF release kinetics from the lead formulations and quantify the fraction of active KGF that was released from the lead formulations.

### EXAMPLES

### Example 1. Preparation and Purification of FITC-Labeled KGF.

10 nmoles of KGF was dissolved in 1 mL of TRIS buffer (pH 8, 10 mM). To this solution was added 10 nmoles of FITC dissolved in the same buffer. Following 8 h of gentle stirring at 4 °C in the dark, unreacted FITC was removed by loading the reaction mixture into a cellulose dialysis bag followed by dialysis against 10 mM TRIS buffer (pH 7.0). The dialysate was replaced with fresh buffer every 8 h until there was no detectable FITC in the dialysate. To ensure the absence of any physisorbed FITC, all KGF-FITC samples were passed through a Sephadex G-25 gel filtration column, eluted with 10 mM TRIS buffer (pH 7.0).

The KGF-FITC purity and the absence of any free FITC was confirmed by capillary electrophoresis (CE). All CE experiments were performed by using a Beckman model P/ACE 2200, Beckman Coulter (Fullerton, California) equipped with a 37 cm long (30 cm to detector) fused silica capillary (50 µm i.d. by 360 µm o.d.), Polymicro Technologies (Phoenix, Arizona). Samples were pressure injected (2-3 s at 5 psi) and separations were carried out at constant voltage (+20 kV) at 25 °C using a 50 mM sodium borate run buffer, pH = 8.5. Laser induced fluorescence detection was performed on column by using the 442 nm output from a He-Cd laser, Series 2056, Omnichrome (Chino, California). Electropherograms for all KGF-FITC adducts were characterized by a single, narrow peak corresponding to FITC-labeled KGF.

### Example 2. Preparation of Biodegradable Polymers.

The following reagents were used: recombinant human KGF (FGF-7) and KGF/FGF-7 antibody; guanidine hydrochloride (GdHCl), methylene chloride, chloroform, *n*-octane, and *iso*-octane, and sodium bis(ethylhexyl)sulfosuccinate (Aerosol-OT, AOT); poly(lactic acid) (PLA, 2 000, 50 000, 100 000, 200 000, and 300 000 MW) and poly(glycolic acid) (PGA); and *n*-heptane.

A series of biodegradable polymer formulations (Table 1 above) were prepared under ambient conditions.

**Table 1. Composition range of KGF-doped biodegradable polymer formulations that were prepared and screened.^{a,b}**

| **PLA^{c}** | **PGA^{d}** | **AOT (mM)** | ***R*^{e}** | **Buffer pH^{f}** |
|---|---|---|---|---|
| 50-100/2K | 0-50 | 3-20 | 0-30 | 6-8 |
| 50-100/50K | 0-50 | 3-20 | 0-30 | 0-30 |
| 50-100/100K | 0-50 | 3-20 | 0-30 | 0-30 |
| 50-100/200K | 0-50 | 3-20 | 0-30 | 0-30 |
| 50-100/300K | 0-50 | 3-20 | 0-30 | 0-30 |

| | | | | |
|---|---|---|---|---|
| (a) Each formulation contains 15 ppm KGF. (b) Each formulation was prepared by using methylene chloride, chloroform, *n*-heptane, *n-*octane, and *iso*-octane as the solvent. (c) Weight percent PLA / Average PLA molecular weight. (d) Weight percent PGA. PGA + PLA = 100% of the total polymer in solution. The total polymer concentration in solution is 3% by weight. (e) [water]/[AOT]. (f) Phosphate or TRIS buffer (12 mM). | | | | |

The biodegradable polymer example formulations were based on PLA, PGA, the surfactant AOT, different water loadings (R = [water]/[AOT]) and pH, and five casting solvents. The typical polymer concentration was 3% by weight in a given solvent. AOT was used in an effort to form reverse micelles into which the KGF would reside and remain stable within the biodegradable formulation. It is not known by the inventors whether reverse micelles were actually formed. There is a body of literature on the use of AOT reverse micelles to stabilize a wide variety of proteins in organic solvents and on the use of surfactants to form drug/protein loaded biodegradable formulations and/or to control formulation biodegradation kinetics. However, to the best of our knowledge, there is no literature proving that reverse micelles such as, for example, AOT can stabilize protein structures within a biodegradable polymer-based formulation and improve protein activity upon liberation from a formulation.

### Example 3. Preparation of Xerogels.

The following reagents were used: mouse monoclonal 4-4-20 anti-fluorescein antibodies and fluorescein (Molecular Probes, Inc.); Dextran (25 000 MW), glycerol, and Nafion (10 wt% dispersion in water) (Aldrich); tetramethyorthosilane (TMOS) and aminopropyl triethoxysilane (APTES) (United Chemical Technologies); and poly(ethyleneimine) (PEE, 70 000 MW, 30 wt% solution in water) and poly(ethyleneoxide) (PEO, 100 000 MW) (Polysciences).

All sol-gel derived solutions (Table 2) were prepared under ambient conditions.

A typical printing solution was prepared by mixing 5 mmoles of silane, 20 mmoles of H₂O, and 0.4x10⁻⁴ mmoles of HCl. After the appropriate hydrolysis time (1 - 24 hrs) each sol was diluted 1:1 (v/v) with buffer. The buffer contained the monoclonal anti-fluorescein antibodies. This solution was mixed for 5 min with a vortex mixer prior to printing.

**Table 2. Composition range of mouse monoclonal 4-4-20 anti-fluorescein antibody-doped sol-gel-derived formulations that were prepared and screened.^{a,b}**

| **TMOS** | **APTES** | **Dextran** | **Nafion** | **PEE** |
|---|---|---|---|---|
| 50-100 | 0-25 | 0-5 | 0-5 | 0-2 |

| **PEO** | **Glycerol** | **Buffer pH^{c}** | **[Buffer] (mM)** | **H.T.^{d}** |
|---|---|---|---|---|
| 0-3 | 0-50 | 6-8 | 1-100 | 1-24 |

| | | | | |
|---|---|---|---|---|
| (a) Each 100 µL aliquot of sol contains 150 pg of anti-fluorescein antibody. (b) Mole percent. (c) Phosphate or TRIS buffer. (e) Hydrolysis time (h) prior to mixing with buffer (1:1). | | | | |

### Example 4. Printing the Formulations for Screening.

All formulations were printed on to fused silica (biodegradable polymers) or glass (xerogels) microscope slides. Individual microscope slides were cleaned first by soaking them in 1 M NaOH for 4 hrs. The slides were subsequently rinsed with copious amounts of deionized water and dried at 80 °C. The liquid formulations were printed directly onto the clean microscope slides by using a ProSys 5510 system (Cartesian Technologies; Irvine, California) with model CMP-3 pins (TeleChem; Sunnyvale, California). The print chamber relative humidity was maintained between 30 and 40%. The final dimensions of each formulation "spot" were a function of the solution composition, relative humidity, pin contact time with the substrate, and substrate's surface chemistry. The individual spots of formulations were 100-150 µm in diameter and they were reproducible to 10% RSD. Scanning electron microscopy showed that the spots of formulations were typically 1-2 µm thick, depending on the exact solution printed, the pin-to-substrate contact time, and the substrate's surface chemistry. The time required to print each formulation was ∼ 1 s. Pins were washed between each formulation by using the ProSys' cleaning program.

All arrays of formulations were allowed to age in the dark at 4 °C for at least three days prior to screening. All spectra and intensity measurements were blank corrected.

### Example 5. Screening to Optimize Composition.

Figure 1 presents a schematic of the apparatus that we constructed to prepare and screen the arrays of formulations. For the anti-fluorescein-doped xerogel studies we used a CW argon-ion laser (Spectra Physics; Mountain View, California, Model 164, λ = 488 nm) as the excitation source 10. For the biodegradable polymer studies we used an intracavity-doubled argon-ion laser (Lexel/Cambridge Lasers; Fremont, , Model 95 SHG, λ = 257 nm) as the excitation source 10. The laser output 15 (10 - 15 mW) was directed to an epi-fluorescence microscope (Olympus; Melville, New York). The laser beam was reflected off the front face of a dichroic filter 20 (Omega; Burlington, Vermont) to a microscope objective 30 (Olympus, 4x) that served to illuminate the array of formulations 40. The resulting luminescence from the array was collected by the microscope objective 30, passed through the dichroic filter 20, and any residual excitation from the laser was suppressed further by an optical filter 25. For the biodegradable polymer studies we were interested in the KGF emission spectra from within each formulation. Given this, we used a series of eight narrow bandpass interference filters 25 (Omega) with transmission maxima spread across the tryptophan emission spectrum. (Note: Because we excite the KGF-doped formulations at 257 nm we could detect emission from the tyrosine and tryptophan residues within the protein.) For the antibody-doped xerogel studies we used a long pass filter 25 (Omega, λ_{cut-on} = 515 nm) because we were only interested in the total emission intensity from anti-fluorescein-bound fluorescein within the formulations. The luminescence 50 from the array of formulations 40 was ultimately imaged on to the face of a thermoelectrically-cooled charge coupled device 60 (CCD, Princeton Instruments; Princeton, New Jersey, Model TE/CCD-1317-K with Model ST-138 controller). The CCD images were acquired, displayed, and processed by using WinView Version 1.6 software (Princeton Instruments) running on a personal computer. The CCD exposure time was typically 0.1 - 0.5 s.

### Example 6. High-Speed Screening Methods Based on Pin-Printing

To maintain the KGF conformation within a biodegradable polymer film we set out to develop a formulation that did not alter the KGF structure within the film. Toward this end, we used a statistical design strategy and prepared a series of blended formulations that were based on a combination of PLA, PGA, the surfactant Aerosol-OT (AOT), and nine casting solvents. Experiments were conducted at least five times using different batches of reagents to yield reliable statistics. We used the AOT to try and form reverse micelles into which the KGF would reside and remain stable and we combined the biodegradable polymers and solvent to produce stable KGF in the films. Initially, we screened 1024 compositions/formulations. (Note: In these experiments we used the intrinsic tryptophan emission from the KGF; no extrinsic fluorescent label is used.) We formed the arrays of formulations using our pin printing methods. Prior to printing we loaded each formulation with an identical concentration of KGF (15 ppm). Each formulation was prepared with different molar ratios of PLA, PGA (poly-glycolic acid), AOT, water/buffer, and casting solvent. We then took this array of 1024 different KGF-doped polymer-based formulations and placed it the apparatus shown in Figure 1. The entire array was illuminated at 285 nm to selectively excite the intrinsic tryptophan residues in the KGF only. All the data from all 1024 formulations were collected simultaneously, passing the emission through an imaging spectrograph to a charge coupled device (CCD) detector. All spectral data for all 1024 formulations was collected in less than 10 min. To assess the stability of the KGF within each formulation, we recorded the emission spectra for all 1024 formulations/spots at regular time intervals. We then analyzed the entire collection of spectra (> 32,000 total) to determine those formulations that exhibited spectra that were the most like native KGF and that did not shift with time. Spectra that are most like native KGF will demonstrate emission maximum and full width at half maximum that are similar to that of native KGF. Six of the 1024 formulations maintained the native KGF conformation. Using these six formulations as leads we prepared another set of 1024 new formulations. The data from this second set of formulations yielded 11 additional formulations that suggested the KGF conformation in the formulations was not different than native KGF. An example of a formulation that yielded a good match to native KGF is composed of 80% PLA + 20% PGA, an AOT concentration of 0.15 M, a molar ratio of buffer-to-surfactant of 11 (pH 7.8) that was cast from chloroform. This particular biodegradable polymer-based formulation was also optically transparent and uniform. These results demonstrate the utility for using our rapid screening method to develop tailored polymer-based architectures in which the protein conformation is not altered significantly within a biodegradable polymer host. These results also prove that we can rapidly screen thousands of polymer-based formulations without difficulty.

### Example 7. Assessing the Formulations.

The KGF-doped biodegradable polymer formulations prepared according to Example 2 were assessed as follows: (1) The entire array was immersed in TRIS buffer (pH 7.4, 10 mM). (2) We recorded the CCD image for the array of formulations using each emission filter (eight images). All data were recorded within 10 min to avoid significant (< 1%) polymer biodegradation or photobleaching (< 3%). (3) From the intensity at each emission wavelength for each formulation we constructed a KGF emission spectrum by fitting the eight data points to a log normal function (PeakFit, Jandel; San Rafael, California). The KGF emission maximum was compared for each formulation to identify formulations that were the most likely to release active KGF after biodegradation of the polymer host matrix. That is, those formulations that yielded KGF emission spectra that were the most similar to KGF dissolved in buffer were considered to be the best performing/most viable. We also performed bioassays on the KGF that was released from several formulations to determine the percentage of the total KGF that was released that was able to bind to KGF/FGF-7 antibodies. This was deemed to be the percentage of active KGF from a given formulation.

The performance of the anti-fluorescein-doped xerogels was assessed as follows: (1) The entire array of xerogel formulations was immersed for 15 min. in 50 mL of 10nM fluorescein dissolved in phosphate buffer (pH 7.4, 10 mM). (2) The entire array was rinsed five times with 50 mL of fresh phosphate buffer to displace any non-specifically adsorbed fluorescein from the xerogels. (3) The intensity from each formulation spot along with its corresponding blank were recorded under identical conditions. Given that the exact same concentration of antibody was present in each formulation and the illumination conditions were constant (2% RSD), those formulations that exhibited the greatest luminescence were the better performing.

We also performed a series of control experiments to determine if there was any non-specific association of fluorescein with inactive antibodies within the xerogels. Toward this end, we replaced the monoclonal anti-fluorescein with monoclonal anti-fluorescein that had been chemically denatured by GdHC1 (8 M). The contribution from non-specific association with inactive antibodies within the xerogels was <6% of the observed emission from a given formulation.

The entire array was prepared in ∼1 hr and the full image was recorded in ∼ 2 min. Two features are readily apparent from an analysis of a false color image of the array of formulations. First, the luminescence from each formulation is different suggesting that the antibodies in each xerogel exhibit different levels of anti-fluorescein affinity within each xerogel. Secondly, the xerogel spots differ in size depending on the formulation composition. Of the 625 formulations that were prepared and screened, most (> 80%) exhibited some detectable degree of antibody affinity (i.e., emission > 5x the blank). An example of a formulation that exhibited a high affinity is the following: 95 % TMOS, 4% APTES, 1% Nafion®, hydrolyisis time = 10 hrs, and pH 6.2 (20 mM) TRIS buffer. Example 8. Determining the Fraction of Active Protein Released form the Biodegradable Polymer-Based Formulations.

We developed a rapid assay to determine active and inactive forms of KGF in solution. Toward this end, we fluorescently-labeled KGF with fluorescein isothiocyanate (FITC) and purified the labeled protein as shown in Example 1. We then performed a standard steady-state fluorescence anisotropy immunoassay by using mouse anti-human KGF as our active KGF-selective ligand. Standard titrations on a set of KGF samples were performed to assess our ability to discriminate and quantify active and inactive KGF. The samples were:
1. A fixed amount of native KGF dissolved in pH 7.5 phosphate buffered saline that had not been subjected to any denaturant. This represents active KGF.
2. A fixed amount of KGF that had been subjected to 1 M GdHCl, with all the GdHC1 removed to assure no denaturation of the anti-human KGF antibody. According to Figure 8A this should also represent active KGF.
3. A fixed amount of KGF that had been subjected to 4 M GdHC1, with all the GdHC1 removed to assure no denaturation of the anti-human KGF antibody, is mixed with an equal molar amount of native KGF. This represents a mixture of active and inactive KGF.

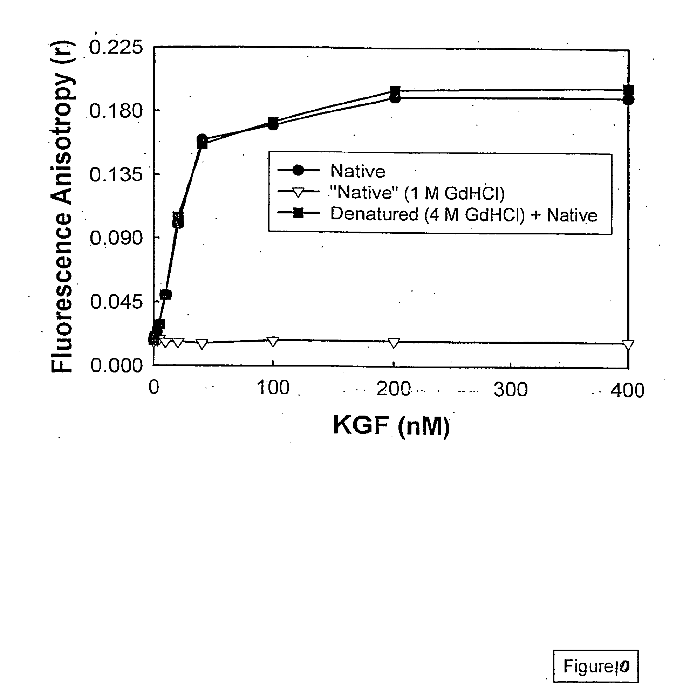

The results of these experiments are summarized in Figure 10. KGF denatured only slightly (Figure 9A) by 1 M GdHC1 is not recognized by the anti-KGF antibodies. This result shows that our assay is remarkably sensitive to slight changes in the KGF conformation. In contrast, the anti-human KGF antibodies readily recognize the native KGF or selectively bind only to the native KGF proteins in a binary mixture that contains known amounts of active and inactive/denatured KGF. Together these results show that we have a simple assay that allows rapid discrimination, in a rigorous fashion, between active and inactive forms of KGF in the supernatant surrounding the biodegradable polymer-based formulations.

### Example 9. Biodegradable Polymeric Formulations.

The data in Table 3 show that KGF released from a PLA film is inactive based on the KGF binding assay. Table 3 compiles kinetic and activity results for the release of KGF from four different biodegradable polymer-based formulations. These particular samples were 6.0 ± 0.2 mm thick films that contained 25 ppm KGF.

**Table 3: Results for KGF in four film formulations (n=5). Release experiments performed in isotonic saline at 35°C.**

| Film Type | Average Release Rate (ng/hr) | % Total KGF Released in 10 hrs | % of Released KGF that is Active |
|---|---|---|---|
| PLA | 24 ± 3 | 1.9 ± 0.1 | 4 ± 4 |
| PLA + AOT^{a} | 39 ± 6 | 2.1 ± 0.2 | 9 ± 4 |
| PLA/PGA^{b} | 83 ± 11 | 19 ± 2 | 12 ± 3 |
| PLA/PGA/AOT^{c} | 94 ± 16 | 27 ± 4 | 58 ± 5 |

| | | | |
|---|---|---|---|
| (a) Average MW = 50 kDa; 0.09 M AOT; molar ratio of water to AOT = 15, solvent = chloroform. (b) 45% PLA (50 kDa); 55% PGA; solvent = chloroform. (c) 80% PLA (20 kDa); 20% PGA; 0.15 M AOT; molar ratio of water (pH 7.8, 0.01 M) to surfactant of 11; solvent = chloroform. | | | |

The KGF activity can be improved slightly by using AOT with PLA (from 4% to 9%). When we used our screening method and investigated blended formulations we found that the combination of PGA and PLA yielded potential lead materials. The data in Table 3 shows that the binary PGA/PLA yielded a biodegradable polymer-based formulation that is superior to any PLA/AOT formulation investigated (12% vs. 9% activity upon release). Finally, when the rapid screening focused our attention on the combination of PLA, PGA, and AOT, we discovered a formulation that released 27% of the KGF loaded into the thin film within 10 hrs and 58% of that KGF that is released from the film is, by the anti-KGF binding assay, active. This is a substantial improvement over the PLA alone and PLA/AOT-based biodegradable formulations.

### Example 10. Choreographed Release of KGF and IL-1 from a Multi-Layer Structure.

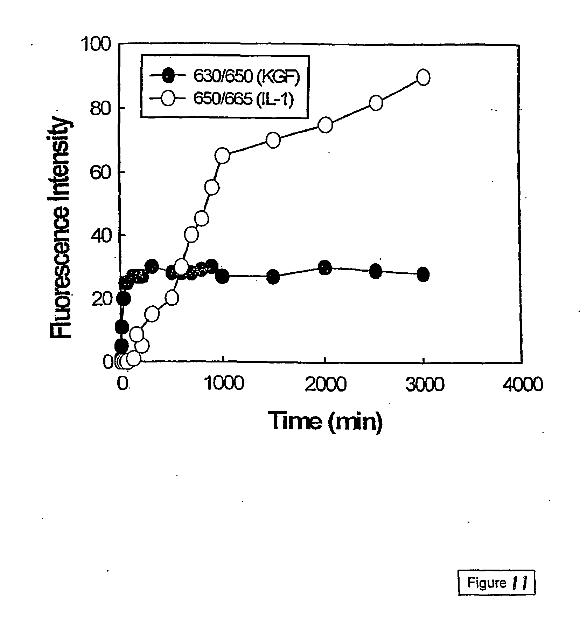

We have also choreographed the release of two different proteins from within a multi-layer polymer-based structure by preparing a two-layer laminate structure by spin casting a 3.7 ± 0.1 mm thick PLA film (60 kDa) onto a fused silica cover slip. We then spun cast, on top of the first layer, a second PLA (60 kDa) film that is 0.4 ± 0.1 mm thick. The thicker layer contained 25 ppm IL-1 (interleukin-1) that we had labeled with the fluorescent probe BODIPY 650/665. This species excites and emits at 650 and 665 nm, respectively. The thinner layer contained 25 ppm KGF that we had labeled with the fluorescent probe BODIPY 630/650. This species excites and emits at 630 and 650 nm, respectively. The protein-doped, two-layer laminated film was immersed in a cuvette containing isotonic saline. Our instrumentation was configured to sequentially excite the solution surrounding the film at 630 nm while monitoring emission at 650 nm and then rapidly switching (< 2 s) to excite at 650 nm while monitoring emission at 665 nm. The film itself was not illuminated during these experiments; only fluorescence from the buffer, arising from liberated/released proteins from the films was measured. In this way we can simultaneously quantify the release of IL-1 and KGF from the two-layer architecture. The results (Figure 11) demonstrate that KGF is released from the two-layer structure first (it is in the top layer). There was no detectable IL-1 released during the KGF release event. IL-1 is only released after the KGF-loaded top film layer has eroded away. At this point, the IL-1 begins to be released as the thicker film starts to erode. The differences in kinetic profiles, save the time lag, arise from differences in film thicknesses and differences in the total number of moles of protein within each film layer. Together these results unequivocally demonstrate choreographed protein release.

### Example 11. Controlling Protein Release Kinetics.

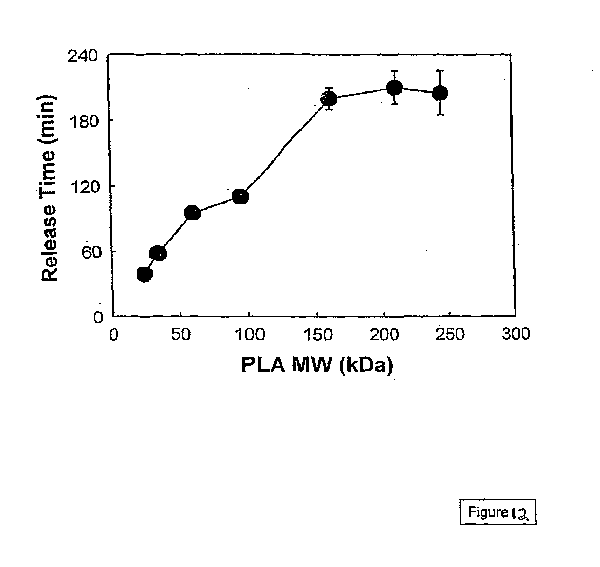

We also developed an additional way to tune the protein release kinetics from our biodegradable polymer-based formulations. (The results in Table 3 show that we can tune the release kinetics by using different components within the formulations and Figure 11 illustrates that one can adjust the amount of protein delivered and the delivery window by adjusting the film thickness.) The results in Figure 12 show that we can also tune the KGF release kinetics from a film of constant thickness (0.4 ± 0.1 mm) by adjusting the average molecular weight of the biodegradable polymer.

## Claims

1. A high throughput method of identifying one or more biomolecule delivery formulations, said method comprising the steps of:
(a) providing a plurality of different test formulations, each formulation prepared by a method comprising:
(i) providing a biomolecule, wherein the biomolecule has an intrinsic luminescence emission spectrum, and wherein the biomolecule is selected from the group consisting of peptides, polypeptides and proteins,
(ii) contacting the biomolecule with a protectant material, wherein the protectant material is a surfactant, and
(iii) sequestening the biomolecule from (ii) in a matrix material, wherein the matrix material is a sol-gel derived glass or a biodegradable polymer;
(b) creating an array of the test formulations;
(c) recording a luminescence emission spectrum for each test formulation in the array;
(d) comparing the luminescence emission spectrum from each test formulation in the array to a luminescence emission spectrum from a control formulation, wherein the control formulation has the biomolecule present in a native conformation;
(e) identifying one or more formulations as biomolecule delivery formulations, based upon comparison of said luminescence emission spectrum from each test formulation to said luminescence emission spectrum from the control formulation;
(f) preparing thin film specimens of each of the one or more candidate biomolecule delivery formulations; and
(g) determining the release kinetics of biologically active labelled biomolecules from each of the one or more thin-film specimens, wherein the release of biologically active labelled biomolecules identifies a biomolecule delivery formulation.

2. The method of Claim 1, wherein the matrix material comprises a biodegradable polymer selected from the group consisting of polyesters, polyanhydrides, polyolefins, polyvinyls, polymethacrylates, polyalkylcyanoacrylates, polyacrylamides, polyorthoesters, polylactones, polycaprolactones, polyphosphazenes, polypeptides, polystyrenes, polyethylenes, polyethers, polyamides, polycarbonates, polyalkylenes, polyurethanes, copolymers thereof and mixtures thereof.

3. The method of Claim 1 or 2, wherein the protectant material comprises a surfactant capable of forming reverse micelles selected from the group consisting a cationic surfactants, anionic surfactants, zwitterionic surfactants, and mixtures thereof.

4. The method of Claim 1 or 2, wherein the protectant material comprises an ionic surfactant selected from the group consisting of sodium oleate, sodium lauryl sulfate, sodium lauryl sarcosinate, sodium dioctyl sulfosuccinate, sodium cholate, sodium taurocholate, lauroyl carnitine, palmitoyl carnitine, myristoyl carnitine, and mixtures thereof.

5. The method of Claim 1 wherein the release kinetics in step (g) are determined by:
(i) exposing the thin-film specimen to a medium such that the specimen degrades;
(ii) analyzing the medium over time; and
(iii) determining the proportion of biologically active labelled biomolecules present in the medium.

6. The method of Claim 5 wherein step (ii) comprises analyzing the degrading medium with an anisotropic immunoassay.

7. The method of Claim 1, wherein the sol-gel derived is a xerogel or an aerogel.

8. The method of Claim 1, wherein the biomolecule is present in the test formulation at a concentration of from 1 to 1000 ppm.

9. The method according to any preceding Claim, wherein said label is a fluorescent tag.

## Patentansprüche

1. Hochdurchsatz-Verfahren zum Identifizieren von einer oder mehreren Biomolekülbereitstellungsformulierungen, wobei das Verfahren die folgenden Schritte umfasst:
(a) das Bereitstellen einer Vielzahl von unterschiedlichen Testformulierungen, wobei jede Formulierung hergestellt wird durch ein Verfahren, umfassend:
(i) das Bereitstellen eines Biomoleküls, wobei das Biomolekül ein Emissionsspektrum intrinsischer Lumineszenz aufweist und wobei das Biomolekül aus der Gruppe ausgewählt ist, welche aus Peptiden, Polypeptiden und Proteinen besteht,
(ii) das Inkontaktbringen des Biomoleküls mit einem Schutzmaterial, wobei das Schutzmaterial ein grenzflächenaktives Mittel ist, und
(iii) das Sequestieren des Biomoleküls von (ii) in einem Matrixmaterial, wobei das Matrixmaterial ein Sol-Gel-abgeleitetes Glas oder ein bioabbaubares Polymer ist;
(b) das Erzeugen eines Arrays von den Testformulierungen;
(c) das Aufzeichnen eines Lumineszenz-Emissionsspektrums für jede Testformulierung in dem Array;
(d) das Vergleichen des Lumineszenz-Emissionsspektrums von jeder Testformulierung in dem Array mit einem Lumineszenz-Emissionsspektrum einer Kontrollformulierung, wobei in der Kontrollformulierung das Biomolekül in einer nativen Konformation vorliegt;
(e) das Identifizieren von einer oder mehreren Formulierungen als Biomolekülbereitstellungsformulierungen, basierend auf einem Vergleich des Lumineszenz-Emissionsspektrums von jeder Testformulierung mit dem Lumineszenz-Emissionsspektrum der Kontrollformulierung;
(f) das Herstellen von dünnen Filmprüfkörpern von jeder der einen oder mehreren potentiellen Biomolekülbereitstellungsformulierungen; und
(g) das Bestimmen der Freisetzungskinetiken von biologisch aktiven markierten Biomolekülen von jedem des einen oder der mehreren dünnen Filmprüfkörpern, wobei die Freisetzung von biologisch aktiven markierten Biomolekülen eine Biomolekülbereitstellungsformulierung identifiziert.

2. Verfahren nach Anspruch 1, wobei das Matrixmaterial ein bioabbaubares Polymer umfasst, welches aus der Gruppe ausgewählt ist, die aus Polyestern, Polyanhydriden, Polyolefinen, Polyvinylen, Polymethacrylaten, Polyalkylcyanoacrylaten, Polyacrylamiden, Polyorthoestern, Polylactonen, Polycaprolactonen, Polyphosphazenen, Polypeptiden, Polystyrolen, Polyethylenen, Polyethern, Polyamiden, Polycarbonaten, Polyalkylenen, Polyurethanen, Copolymeren davon und Gemischen davon besteht.

3. Verfahren nach Anspruch 1 oder 2, wobei das Schutzmaterial ein grenzflächenaktives Mittel umfasst, welches zum Bilden von inversen Mizellen in der Lage ist und aus der Gruppe ausgewählt ist, welche aus kationischen grenzflächenaktiven Mitteln, anionischen grenzflächenaktiven Mitteln, zwitterionischen grenzflächenaktiven Mitteln und Gemischen davon besteht.

4. Verfahren nach Anspruch 1 oder 2, wobei das Schutzmaterial ein ionisches grenzflächenaktives Mittel umfasst, welches aus der Gruppe ausgewählt ist, die aus Natriumoleat, Natriumlaurylsulfat, Natriumlaurylsarcosinat, Natriumdioctylsulfosuccinat, Natriumcholat, Natriumtaurocholat, Lauroylcarnitin, Palmitoylcarnitin, Myristoylcarnitin und Gemischen davon besteht.

5. Verfahren nach Anspruch 1, wobei die Freisetzungskinetiken in Schritt (g) bestimmt werden durch:
(i) das Einwirken eines Mediums auf den dünnen Filmprüfkörper, so dass der Prüfkörper abgebaut wird;
(ii) das Analysieren des Mediums im Verlauf der Zeit; und
(iii) das Bestimmen des Anteils von biologisch aktiven markierten Biomolekülen, welche in dem Medium vorhanden sind.

6. Verfahren nach Anspruch 5, wobei der Schritt (ii) das Analysieren des Abbaumediums mit einem anisotropen Immunotest umfasst.

7. Verfahren nach Anspruch 1, wobei das Sol-Gel-abgeleitete Material ein Xerogel oder ein Aerogel ist.

8. Verfahren nach Anspruch 1, wobei das Biomolekül in der Testformulierung in einer Konzentration von 1 bis 1000 ppm vorhanden ist.

9. Verfahren gemäß einem vorhergehenden Anspruch, wobei die Markierung ein fluoreszentes Tag ist.

## Revendications

1. Procédé à haut débit pour l'identification d'une ou de plusieurs formulations permettant la délivrance de biomolécules, ledit procédé comprenant les étapes consistant à :
(a) fournir une pluralité de formulations de test différentes, chaque formulation étant préparée par un procédé comprenant :
(i) la fourniture d'une biomolécule, la biomolécule ayant un spectre d'émission de luminescence intrinsèque, et la biomolécule étant choisie dans le groupe constitué par les peptides, les polypeptides et les protéines,
(ii) la mise en contact de la biomolécule avec un matériau protecteur, le matériau protecteur étant un surfactant, et
(iii) la séquestration de la biomolécule de l'étape (ii) dans un matériau de matrice, le matériau de matrice étant un verre obtenu par un procédé sol-gel ou un polymère biodégradable ;
(b) créer une rangée des formulations de test ;
(c) enregistrer un spectre d'émission de luminescence pour chaque formulation de test dans la rangée ;
(d) comparer le spectre d'émission de luminescence de chaque formulation de test dans la rangée à un spectre d'émission de luminescence d'une formulation de contrôle, la biomolécule étant présente dans la formulation de contrôle dans une conformation native ;
(e) identifier une ou plusieurs formulations en tant que formulations permettant la délivrance de biomolécules, en se basant sur la comparaison dudit spectre d'émission de luminescence de chaque formulation de test au dit spectre d'émission de luminescence de la formulation de contrôle ;
(f) préparer des échantillons minces de la ou de chacune des formulations candidates permettant la délivrance de biomolécules ; et
(g) déterminer les cinétiques de libération de biomolécules biologiquement actives marquées du ou de chacun des échantillons minces, la libération de biomolécules biologiquement actives marquées identifiant une formulation de délivrance de biomolécules.

2. Procédé selon la revendication 1, dans lequel le matériau de matrice comprend un polymère biodégradable choisi dans le groupe constitué par les polyesters, les polyanhydrides, les polyoléfines, les polyvinyles, les polyméthacrylates, les polyalkylcyanoacrylates, les polyacrylamides, les polyorthoesters, les polylactones, les polycaprolactones, les polyphosphazènes, les polypeptides, les polystyrènes, les polyéthylènes, les polyéthers, les polyamides, les polycarbonates, les polyalkylènes, les polyuréthanes, leurs copolymères et leurs mélanges.

3. Procédé selon la revendication 1 ou 2, dans lequel le matériau protecteur comprend un surfactant capable de former des micelles inverses choisies dans le groupe constitué par les surfactants cationiques, les surfactants anioniques, les surfactants zwitterioniques, et leurs mélanges.

4. Procédé selon la revendication 1 ou 2, dans lequel le matériau protecteur comprend un surfactant ionique choisi dans le groupe constitué par l'oléate de sodium, le lauryl sulfate de sodium, le lauryl sarcosinate de sodium, le dioctyl sulfosuccinate de sodium, le cholate de sodium, le taurocholate de sodium, la lauroyl carnitine, la palmitoyl carnitine, la myristoyl carnitine, et leurs mélanges.

5. Procédé selon la revendication 1, dans lequel les cinétiques de libération dans l'étape (g) sont déterminées par :
(i) exposition de l'échantillon mince à un milieu tel que l'échantillon se dégrade ;
(ii) analyse du milieu dans le temps ; et
(iii) détermination de la proportion de biomolécules biologiquement actives marquées présentes dans le milieu.

6. Procédé selon la revendication 5, dans lequel l'étape (ii) comprend l'analyse du milieu de dégradation avec un dosage immunologique anisotrope.

7. Procédé selon la revendication 1, dans lequel le verre obtenu par un procédé sol-gel est un xérogel ou un aérogel.

8. Procédé selon la revendication 1, dans lequel la biomolécule est présente dans la formulation de test en une concentration comprise entre 1 et 1000 ppm.

9. Procédé selon l'une quelconque des revendications précédentes, dans lequel ledit marqueur est un marqueur fluorescent.
